# EUROPÄISCHE PATENTANMELDUNG

(11) **EP 0 610 555 A2**
(43) Veröffentlichungstag der Anmeldung: **17.08.1994**
(21) Anmeldenummer: 93118185.3
(22) Anmeldetag: 10.11.1993
(51) Int. Cl.: G01F 1/00, G01N 15/08

(54) **Vorrichtung zur Bestimmung der Luftdurchlässigkeit einer Warenbahn**

(30) Priorität: 09.02.1993 CH 389/93
(71) Anmelder: TEXTEST AG, CH-8051 Zürich (CH)
(72) Erfinder: Vogt, Horst, CH-8044 Zürich (CH)
(74) Vertreter: Hunziker, Jean

(57) **Zusammenfassung**

Für die kontinuierliche Messung der Luftdurchlässigkeit an bewegten oder stillstehenden Warenbahnen (B) wird diese über eine Messöffnung (9) geführt, die am Ende eines Messrohres (8) in die konvex gekrümmte Aussenfläche eines Messkopfes (5) mündet. Diese konvex gekrümmte Fläche lenkt die Bahn (B) aus. Durch die Zugspannung im Bahnmaterial schmiegt sich die Bahn (B) dadurch selbsttätig um die Messöffnung (9) herum dichtend an diese Fläche. Im Messrohr (8) wird durch eine Pumpe (10) Luft durch den über der Messöffnung (9) liegenden Bahnteil angesaugt. Die Strömungsgeschwindigkeit der durch das Prüfmuster gesaugten Luft wird in einem Durchflussmesser (15) gemessen und als Luftdurchlässigkeit des Prüfmusters von einem Anzeigegerät (16) angezeigt und gegebenenfalls zur Regelung verwendet wird.

## Beschreibung

Die Erfindung bezieht sich auf eine Vorrichtung zur Bestimmung der Luftdurchlässigkeit einer Warenbahn nach dem Oberbegriff des Patentanspruchs 1.

Vorrichtungen dieser Art finden Anwendung für die schnelle und genaue Bestimmung der Luftdurchlässigkeit von flächigen Gebilden aller Art, z.B. von Textilbahnen zur Herstellung von Ballonhüllen, Fallschirmen, Geotextilien aber auch Drahtgittern usw. im Labor oder direkt bei oder während der Produktion oder Behandlung solcher Warenbahnen. Da die Luftdurchlässigkeit ein Massstab für die Dichte eines Flächengebildes bildet, kann die Bestimmung der Luftdurchlässigkeit auch direkt zur Erkennung von Strukturänderungen in der überprüften Warenbahn bei gezogen werden.

Eine bekannte Vorrichtung der genannten Gattung umfasst einen Messkopf mit einem zylindrischen Messrohr, das zur Bildung einer zweckmässig kreisrunden Messöffnung in eine Fläche des Messkopfes mündet. Mit dem Messrohr ist die Saugseite einer Pumpe verbunden. Zur Bestimmung der Luftdurchlässigkeit wird ein Prüfmuster der Warenbahn zwischen die Messöffnung und einen auf die Grösse der Messöffnung abgestimmten Andruckring gepresst und mit der Pumpe Luft durch die Messöffnung, das eingeklemmte Prüfmuster und den Andruckring gesaugt. Die Luftdurchlässigkeit ist die Geschwindigkeit des Luftstromes bei einem gegebenen Druckabfall am Prüfmuster. Die Strömungsgeschwindigkeit der durch das Prüfmuster gesaugten Luft wird in einem Durchflussmesser gemessen und als Luftdurchlässigkeit des Prüfmusters von einem Anzeigegerät angezeigt.

Diese bekannte Vorrichtung ist aber für Messungen an einem stillstehenden Prüfmuster konzipiert. Für eine Bestimmung der Luftdurchlässigkeit an einer laufenden Bahn ist sie nicht geeignet. Denn dazu müssten der Messkopf zusammen mit dem Andruckring synchron mit der laufenden Bahn bewegt werden, weil eine einwandfreie Abdichtung am Umfang der Messöffnung mittels eines stationären Andruckrings bei einer laufenden Bahn nicht möglich ist. Das würde aber zu sehr aufwendigen Konstruktionen führen. Anderseits wäre eine Bestimmung der Luftdurchlässigkeit an einer laufenden Bahn, z.B. in einer Behandlungsanlage, in welcher eine Bahn in ausgebreitetem Zustand unter einer geregelten Spannung kontinuierlich hindurchgeführt wird, sehr erwünscht, da dadurch ein Stillsetzen der Anlage zum Zwecke der Messung der Luftdurchlässigkeit vermieden werden könnte.

Mit der Erfindung wird die Aufgabe gelöst, eine Vorrichtung der eingangs genannten Gattung zu schaffen, welche, bei einfachstem Aufbau, eine einwandfreie Messung der Luftdurchlässigkeit einer ausgebreitet transportierten Warenbahn ermöglicht.

Die Lösung dieser Aufgabe ist im kennzeichnenden Teil des Anspruchs 1 angegeben.

Durch diese einfache Massnahme erfolgt die notwendige Seitenabdichtung um die Messöffnung herum automatisch und zwar auch bei laufender Bahn, sodass die Notwendigkeit eines speziellen Andruckringes entfällt. Mit der erfindungsgemässen Vorrichtung kann die Luftdurchlässigkeit auch einer laufenden Bahn bestimmt werden, da es genügt, mindestens einen Teil der Bahnbreite über den Messkopf zu führen, der an einer geeigneten Stelle des Bahnlaufs stationär angeordnet sein kann.

Die Aussenfläche des Messkopfes kann kugelige oder halbzylindrische Form aufweisen und so angeordnet sein, dass sie die Bahn auf deren Weg über diese Fläche umlenkt oder aus ihrer Transportebene auslenkt um hierdurch die Abdichtung um die Messöffnung herum zu erzielen.

Mit den Massnahmen nach den Ansprüchen 3 bis 6 kann die erforderliche Abdichtung noch weiter verbessert werden.

Die Weiterbildung nach Anspruch 7 erlaubt es, die Luftdurchlässigkeit einer Bahn gleichzeitig an mehreren Stellen der Bahnbreite zu bestimmen.

Mit der Massnahme nach Anspruch 9 wird eine konstruktive Vereinfachung des Messkopfes erreicht, und weil das Prüfmuster völlig eben auf der Messöffnung aufliegt und die Luft über die ganze Fläche des Prüfmusters senkrecht zu diesem hindurchgesogen wird, wird die Messgenauigkeit erhöht..

Die Erfindung wird im folgenden anhand von Ausführungsbei spielen mit Bezug auf die schematischen Darstellungen in der Zeichnung näher erläutert. Es zeigt:
- Fig. 1: eine besonders einfache Ausführungsform einer erfindungsgemässen Vorrichtung und die Führung einer ausgebreitet transportierten Warenbahn vor und hinter dieser Vorrichtung;
- Fig. 2: in einer schematischen Darstellung analog derjenigen der Fig. 1 eine zweite Ausführungsform einer erfindungsgemässen Vorrichtung;
- Fig. 3: eine Variante der Ausbildung des Messkopfes;
- Fig. 4: eine weitere Variante der Ausbildung des Messkopfes, und
- Fig. 5: in einer Draufsicht auf den Messkopf ein weiteres Ausführungsbeispiel einer erfindungsgemässen Vorrichtung.

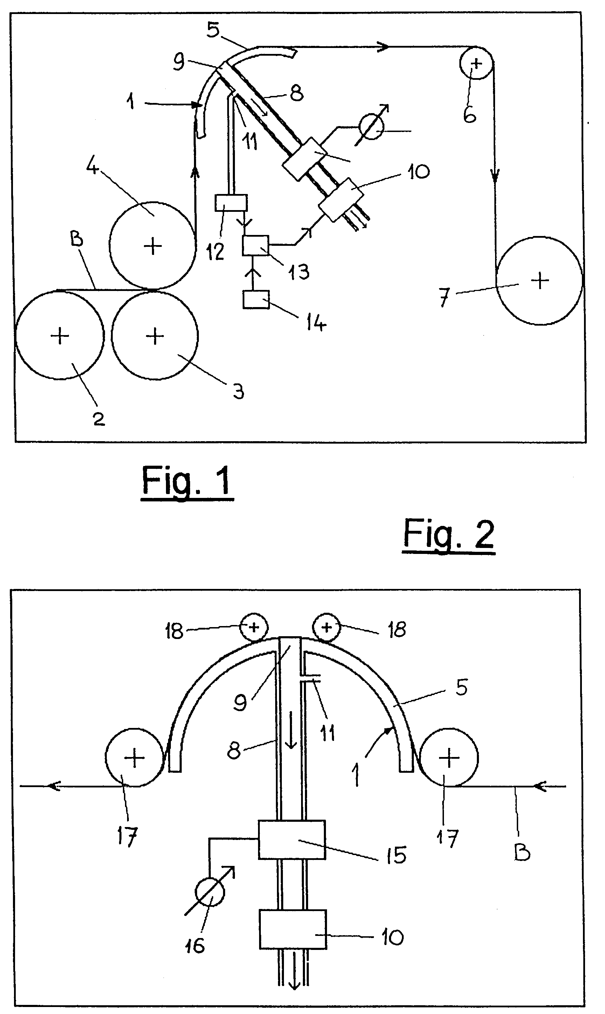

Die Fig. 1 zeigt die Anordnung einer Vorrichtung zum Bestimmen der Luftdurchlässigkeit in einem schematisch dargestellten Kalander. Die auf einer Vorratstrommel 2 gewickelte Warenbahn B wird von dieser abgewickelt, zwischen einem Walzenpaar 3,4 hindurchgeführt und umgelenkt und gelangt dann über eine weitere Umlenkwalze 6 zu einer Aufwickeltrommel 7, auf welche die Bahn B wieder aufgewickelt wird. Auf ihrem ganzen Weg von der Vorratstrommel 2 zu der Aufwickeltrommel 7 wird die Bahn B ausgebreitet geführt und in bekannter Weise unter einer geregelten Zugspannung gehalten. Zwischen der Umlenkung durch die Walze 4 des Walzenpaares 3,4 und der Umlenkwalze 6 wird die Warenbahn B ein weiteres Mal umgelenkt, und zwar über die äussere, quer zur Längsachse der Bahn B konvex gekrümmte Fläche des Messkopfes 5 einer allgemein mit 1 bezeichneten Vorrichtung zur Bestimmung der Luftdurchlässigkeit der Warenbahn B. Von der der Bahn B abgewendeten Seite des Messkopfes 5 mündet ein Messrohr 8 in die Aussenfläche des Messkopfes 5 und bildet eine Messöffnung 9.Im Betrieb der Vorrichtung 1 wird mit Hilfe einer Pumpe 10 Luft in Pfeilrichtung durch den die Messöffnung 9 abdeckenden Teil der Warenbahn B gesaugt. Der Druckabfall an der Warenbahn B wird an einer Druckmessstelle 11 im Messrohr 8 abgenommen und über eine Leitung einem Drucksensor 12 zugeführt. Das Ausgangssignal des Drucksensors 12 wird in einem Regelverstärker 13 mit einem einstellbaren Sollwert verglichen, den ein Sollwertgeber 14 liefert. Das Differenzsignal entsprechend Istwert minus Sollwert wirkt auf die Pumpe 10 zurück, sodass der Druckabfall an der Warenbahn B auf dem eingestellten Sollwert konstantgehalten wird.

Die Strömungsgeschwindigkeit der durch den auf die Messöffnung aufliegenden Teil der Bahn B gesogenen Luft wird in einem Durchflussmesser 15 gemessen und in einem Anzeigegerät 16 als Luftdurchlässigkeit der Bahn B angezeigt und gegebenenfalls zur Steuerung der Anlage beigezogen.

Die in der laufenden Warenbahn B aufrechterhaltene Zugspannung sorgt jederzeit für ein enges Anliegen der Bahn B auf der konvexen Aussenfläche des Messkopfes 5 und dadurch für eine gute Abdichtung zwischen der Bahn B und der genannten Aussenfläche rings um die Messöffnung 9.

Beim Ausführungsbeispiel gemäss Figur 2 wird die Warenbahn B vor und hinter dem Messkopf 5 über je eine Umlenkwalze 17 geführt. Dadurch wird bei einer eben geführten Warenbahn B ein genügend grosser Umschlingungswinkel der Bahn B um den Messkopf erreicht. Bei den Umlenkwalzen 17 kann es sich um frei laufende Walzen handeln oder aber um gleichsinnig, gegensinnig mit gleicher oder unterschiedlicher Geschwindigkeit angetriebene, Walzen um eine Regelung der Zugspannung der Bahn B und damit der Andruckkraft, mit welcher die Bahn zur Erzielung der erforderlichen Abdichtung an den Messkopf angedrückt wird, zu verändern. Mit Hilfe weiterer Presswalzen 18, die, in Längsrichtung der Bahn B gesehen, unmittelbar vor, hinter und/oder neben der Messöffnung 9 angeordnet sind, kann der Andruck der Bahn B an den Messkopf nochmals gesteigert werden.

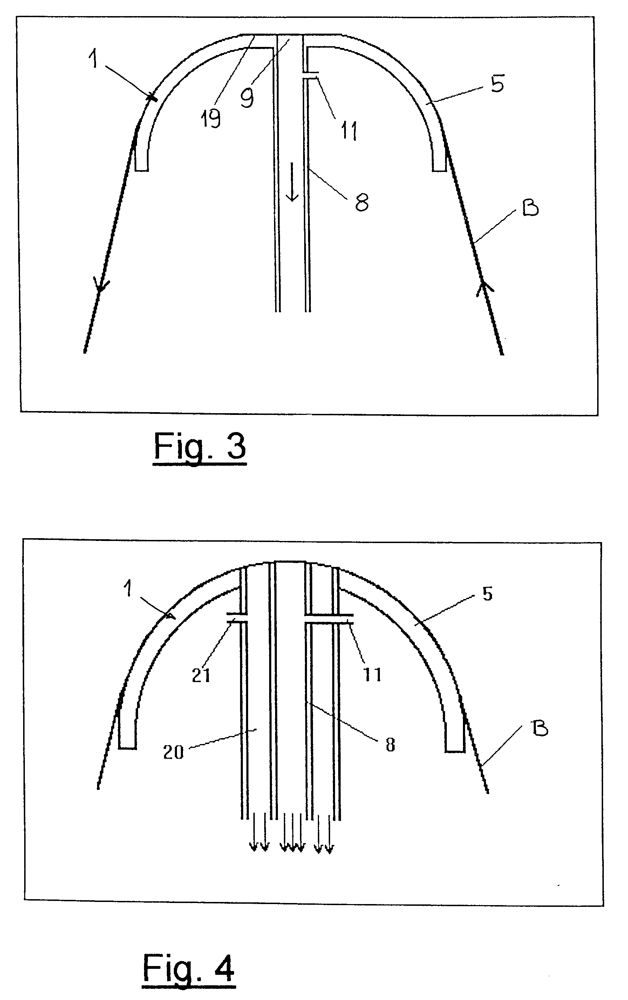

In der in Figur 3 dargestellten Variante weist die Aussenfläche des Messkopfes 5 im unmittelbaren Nahbereich der Messöffnung 9 quer zur Längsachse der Bahn B gesehen einen ebenen Mittelteil 19 auf. Dadurch wird eine konstruktive Vereinfachung des Messkopfes 5 erreicht. Da die Warenbahn B völlig eben auf der Messöffnung 9 liegt, strömt die Luft im gesamten Bereich der Messöffnung senkrecht durch die Bahn B, so dass das Messergebnis nicht durch Winkelfehler verfälscht werden kann.

Bei der Ausführungsform gemäss Figur 4 ist das Messrohr 8 von einem konzentrisch dazu verlaufenden Ringkanal 20 umgeben, der ebenfalls konzentrisch zur Messöffnung 9 in die konvex gekrümmte Aussenfläche des Messkopfes mündet. Mit Hilfe einer, hier nicht dargestellten, zweiten Pumpe wird durch diesen Ringkanal 20 soviel Luft angesaugt, dass in diesem Ringkanal 20 der gleiche Unterdruck herrscht wie im Messkanal 8. Dadurch wird weitgehend verhindert, dass von der Seite her Luft in das Messrohr 8 eindringen und die Messung verfälschen kann.

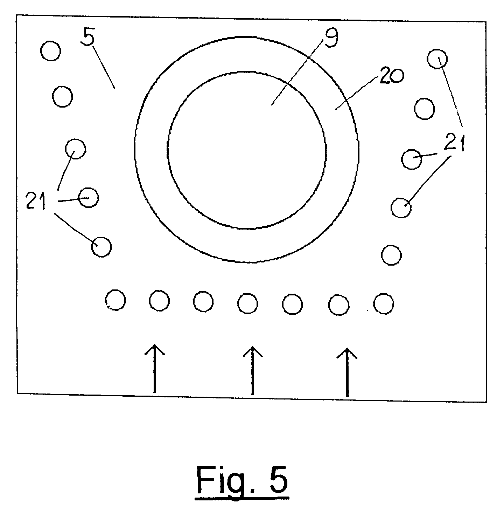

In Figur 5 schliesslich ist in einer Draufsicht auf den Messkopf 5 eine Weiterbildung der Ausführungsform nach Figur 4 gezeigt. In dieser Ausführung weist die Vorrichtung, zusätzlich zu dem das Messrohr umgebenden, konzentrisch zur Messöffnung 9 in die Aussenfläche des Messkopfes mündenden Ringkanal 20, eine Reihe von Löchern 21 oder von Schlitzen auf, die dafür sorgen, dass allfällig zwischen der Bahn B und dem Messkopf 5 eingeschlossene Luft abfliessen kann, bevor die Bahn B die Messöffnung 9 erreicht. Um das Anliegen der Bahn B am Messkopf 5 noch weiter zu verbessern kann mit Hilfe einer Pumpe in diesen Bohrungen 21 oder Schlitzen ein Unterdruck erzeugt werden, der die Bahn B an die Aussenfläche des Messkopfes 5 ansaugt.

Alle beschriebenen Ausführungen der erfindungsgemässen Vorrichtung können, in hier nicht näher dargestellten Weise, noch Mittel aufweisen, um zusätzlich zur Bestimmung der Luftdurchlässigkeit der Bahn B auch deren Dicke zu bestimmen.

Schliesslich ist es auch ohne weiteres möglich, die erfindungsgemässe Vorrichtung so auszubilden, dass sie, quer zur Bahnlaufrichtung gesehen, mehrere unter sich nach einer der vorstehend beschriebenen Ausführungsformen gleich ausgebildete Messanordnungen aufweist, um die Luftdurchlässigkeit der Bahn B gleichzeitig an mehreren Stellen der Bahnbreite zu bestimmen.

## Patentansprüche

1. Vorrichtung zur Bestimmung der Luftdurchlässigkeit einer zwischen Walzen ausgebreitet gespannt gehaltenen flächigen Warenbahn, mit einem zur Bildung einer Messöffnung in eine Fläche eines Messkopfes mündenden Messrohr, mit Mitteln, um einen über die Messöffnung geführten Bereich der Warenbahn am Umfang der Messöffnung luftdicht abzudichten, einer Pumpe zur Erzeugung eines Unterdruckes im Messrohr und Mitteln zur Bestimmung der durch die Warenbahn gesogenen Luftmenge, dadurch gekennzeichnet, dass die genannte Fläche des Messkopfes (5) im Bereich der Messöffnung (9) konvex gekrümmt ist und eine Auslenkung der Warenbahn (B) bewirkt, durch welche letztere selbsttätig um die Messöffnung (9) herum dichtend an die genannte Fläche gepresst ist.

2. Vorrichtung nach Anspruch 1, dadurch gekennzeichnet, dass die Aussenfläche des Messkopfes (5) kugelige oder halbzylindrische Form aufweist und so angeordnet ist, dass sie die Bahn auf deren Weg über diese Fläche umlenkt oder aus ihrer Transportebenen auslenkt.

3. Vorrichtung nach Anspruch 1 oder 2, dadurch gekennzeichnet, dass im Messkopf (5) ein Ringkanal (20) konzentrisch um die Messöffnung (9) herum angeordnet ist, in welchem im Betrieb der gleiche Unterdruck herrscht wie im Messrohr an der Messöffnung. (Fig.4)

4. Vorrichtung nach einem der vorangehenden Ansprüche, dadurch gekennzeichnet, dass mindestens in Laufrichtung der Bahn (B) vor der Messöffnung (9) Bohrungen (21) oder Schlitze angeordnet sind, um zwischen der Bahn (B) und dem Messkopf (5) eingeschlossene Luft vor der Messung abzuleiten. (Fig. 5)

5. Vorrichtung nach Anspruch 4, dadurch gekennzeichnet, dass in den genannten Bohrungen (21) oder Schlitzen ein Unterdruck herrscht, der die Bahn zusätzlich an die Messkopffläche ansaugt.

6. Vorrichtung nach einem der vorangehenden Ansprüchen, dadurch gekennzeichnet, dass in Bahnlaufrichtung gesehen, vor, neben und/oder hinter der Messöffnung (9) Walzen (17,18) angeordnet sind, welche die Bahn (B) an die Messkopffläche drücken. (Fig. 2)

7. Vorrichtung nach einem der vorangehenden Ansprüche, dadurch gekennzeichnet, dass sie, quer zur Bahnlaufrichtung gesehen, mehrere unter sich gleich ausgebildete Messanordnungen aufweist, um die Luftdurchlässigkeit der Bahn gleichzeitig an mehreren Stellen der Bahnbreite zu bestimmen.

8. Vorrichtung nach einem der vorangehenden Ansprüche, dadurch gekennzeichnet, dass sie Mittel aufweist, um zusätzlich zur Bestimmung der Luftdurchlässigkeit auch die Dicke der Bahn zu messen.

9. Vorrichtung nach einem der vorangehenden Ansprüche, dadurch gekennzeichnet, dass die Aussenfläche des Messkopfes (5) im unmittelbaren Nahbereich der Messöffnung quer zur Längsachse der Bahn (B) eben ist. (Fig. 3)
